# EUROPEAN PATENT SPECIFICATION

(11) **EP 1 575 520 B1**
(45) Date of publication and mention of the grant of the patent: **07.11.2007**
(21) Application number: 03811861.8
(22) Date of filing: 03.09.2003
(51) Int. Cl.: C12Q 1/04, G01N 33/569

(54) **A PROCESS FOR PREPARATION OF AN AGGLUTINATION REAGENT FOR RAPID DETECTION OF TYPHOID**
VERFAHREN ZUR HERSTELLUNG EINES AGGLUTINATIONSREAGENS FÜR DIE SCHNELLBESTIMMUNG VON TYPHUS
PROCEDE DE PREPARATION D`UN REACTIF D`AGGLUTINATION DES TINE A LA DETECTION RAPIDE DE LA TYPHOIDE

(30) Priority: 26.11.2002 IN DE11872002; 12.03.2003 IN DE02712003
(43) Date of publication of application: 21.09.2005
(73) Proprietor: Defense Research & Development Organisation, New Delhi 110 011 (IN)
(72) Inventor: RAI, Ganga, Prasad, Gwalior 474 002 (IN); AGARWAL, Gauri, Shankar, Gwalior 474 002 (IN); SHARMA, Shri, Krishna, Gwalior 474 002 (IN); JAISWAL, Devendra, Kumar, Gwalior 474 002 (IN); SHEKHAR, Krishanamurthy, Gwalior 474 002 (IN); ARORA, Kajal C/o Deptt. of Biochemistry, New Delhi 110 002 (IN); CHAUDHARY, Vijay Kumar C/o Deptt. of Biochemistry, New Delhi 110 002 (IN)
(74) Representative: Brommer, Hans Joachim
(86) International application number: PCT/IN2003/000296
(87) International publication number: WO 2004/047721

(56) References cited:
- EP-A1- 0 599 803
- WO-A1-96/31777
- US-A- 4 960 715
- DATABASE CA DATABASE [Online] LIM P.L. ET AL: 'A one-step two-particle latex immunoassay for the detection of Salmonella typhi endotoxin.', XP002992592 Retrieved from STN-international. Database accession no. (114:137509) & JOURNAL OF IMMUNOLOGICAL METHODS. vol. 135, no. 1-2, 1990, pages 257 - 261

## Description

### FIELD OF INVENTION

The present invention relates to a process for the preparation of an agglutination reagent for rapid and early detection of Salmonella typhi in serum.

### PRIOR ART

A process for the preparation of an agglutination reagent for rapid and early detection of typhoid comprising the features of the upper part of claim 1 is disclosed in US-A-4960715 and in Lim & Yoy (1988), J. Immunol. Methods, 115,269-274.

Typhoid is an endemic febrile disease caused by Salmonella typhi. Typhoid is a major concern of public health. The organism usually enters the body by consumption of contaminated food or water and penetrates the intestinal wall. After that it multiplies and enters blood stream within 24-72 hours resulting in enteric fever and bacteremia. After an incubation period of 10 to 14 days, early symptoms of typhoid, like headache, fever, loss of appetite, bradycardia, splenomegaly etc. appear. Typhoid is diagnosed either by blood culture or by detection of its antigens or by the detection of its antibodies in the blood.

One of the most adapted methods for diagnosing the typhoid fever is the performance of "Widal test", a serological test based on the detection of antibodies in the blood. This test in based on the fact that antibodies against typhoid, remain in the blood of infection person, bind to the bacteria and results in the clumps formation which is referred as "Widal Agglutination"

One of the limitation of the widal test is that the test is not specific as it cross reacts with other febrile organisms and many organisms of family Enterobacteriaceae.

Another limitation of the widal test is that, as typhoid is an endemic disease hence there always exist some background level of antibody in the endemic areas. Hence it becomes necessary to determine the cut-off titra for each region to rule out the possibility of diagnosis as false positive.

Yet another limitation of the widal test is that it gives position results only after one or two weeks of the onset of fever.

Still another limitation of the widal test is that test it is to be performed on paired serum samples taken at an interval of at least one week apart because single widal test is elusive and inconclusive.

Further limitation of the widal test is that the antibiotic administration in the early phase of infection, inhibits the development of the antibody and hence test may give false negative result.

Still further limitation of the widal test is that TAB vaccinated normal healthy persons give false positive reaction in widal test due to presence of circulating antibody against vaccine in human system.

Another limitation of the widal test is that it gives indirect evidence of typhoid infection.

Further limitation of the widal test is that the test has low sensitivity and low specificity.

Other technique known for diagnosis of typhoid is based upon isolation and identification of causative agent. This procedure is termed as golden standard.

In this technique Salmonella typhi is isolated from blood and identified by microscopic and biochemical tests. However, this technique has many limitations.

One limitation of the above technique is that it is time consuming as it requires long period of incubation from 3 days to 14 days and also requires elaborate laboratory facilities.

Another limitation of the above technique is that for its performance large quantity of blood sample (10 ml/patient) is required.

Yet another limitation of the above technique is that it needs large volume of culture medium i.e. 100 ml (10 times of blood sample).

Still another limitation of the above technique is its low sensitivity (40 to 60%), as there are very few organism in circulation, as low as 1/ml which leads to false negative results.

Further limitation of above method is that bacterial growth in culture is inhibited by serum bactericidal agents, present in blood which may lead to false negative results.

Still further limitation of blood culture is that antibiotics treatment during early phase of infection may inhibit bacterial growth in culture which may give false negative results.

Other known techniques such as Radioimmunoassay (RIA), Enzyme - linked immunosorbent assay etc. are based on detection of circulating antigen in the body fluids, but these techniques have many limitations.

One limitation of these techniques is that they require sophisticated and elaborate laboratory facilities.

Another limitation of RIA is that it requires radioactive material which is health hazard and also needs trained personnel to handle the radioactive material.

Still further limitation of above techniques is that reagents are expensive.

Further limitation of these techniques is that minimum 4-5 hours are required to perform the tests.

### OBJECT OF THE INVENTION

The primary object of the present invention is to provide a process for the preparation of an agglutination reagent for rapid and early detection of S.typhi in serum samples of suspected typhoid patients.

Another object of the present invention is to provide a process for the preparation of an agglutination reagent wherein the proposed reagent enables diagnosis of typhoid within 3 minutes after collection of serum samples:

Yet another object of the present invention is to provide a process for the preparation of an agglutination reagent wherein the serum sample required for the diagnosis of typhoid disease is as small as 20 µl.

Further object of the present invention is to provide a process for the preparation of an agglutination reagent wherein the reagent enables the detection of typhoid bacteria by simple latex agglutination technique.

Still further object of the present invention is to provide a process for the preparation of an agglutination reagent wherein the reagent enables specific identification of Salmonella typhi antigen in serum samples of suspected typhoid patients.

Yet further object of the present invention is to provide a process for the preparation of an agglutination reagent wherein the reagent enables the diagnosis of typhoid in the early stages of infection even within one or two days after the on set of the fever.

Another object of the present is to provide a process for the preparation of an agglutination reagent wherein the reagent is highly sensitive.

Yet another object of the present invention is to provide a process for the preparation of an agglutination reagent which enables diagnosis of the disease in field conditions as it does not require any equipment or laboratory facility.

Still further object of the present invention is to provide a process for the preparation of an agglutination reagent that does not require any specially trained personnel to perform the test.

Yet further object of the present invention is to provide a process for the preparation of an agglutination reagent which enables the diagnosis of those patients who have been administered with antibiotics resulting in blood culture isolation as negative.

### DESCRIPTION OF PROCESS

According to the present invention, the agglutination reagent is prepared by a process comprising the features of claim 1.

### (a) Preparation of antibody (immunoglobulins)

Flagellin gene sequence specific to Salmonella typhi is cloned and expressed by recombinant DNA technology. The expressed recombinant protein is purified by affinity chromatography. Hyper immune sera against this recombinant protein is raised in rabbit. Immunoglobulin fraction of hyper immune sera is separated by ammonium sulphate precipitation. The precipitation immunoglobulins in 50mM phosphate buffer (pH 7.2), dialysed and protein content determined.

### (b) Preparation of Latex Particles suspension:

1% carboxylated latex particles of size 0.88 to 0.90 µm and 40mM 2-N Morphilinoethane sulphonic acid (MES) buffer (pH 5.5-6.0) are taken In a preferred ratio of 1:1 in a tube. They are mixed on a vortex mixer for around 60 seconds and centrifuged at 10,000 rpm for 10-12 minutes at about 4°C. The latex particles are further washed twice in 20mM MES buffer of pH 5.5 by mixing on vortex mixer for around 60 seconds, followed by centrifugation at 10,000 rmp for 10-12 minutes at about 4°C. After the final wash, the latex particles are suspended In 20mM MES buffer of pH 5.5 and the volume is made up equal to the starting volume of the latex particles. The suspension is then sonicated by a tip sonicator at about 5 watts for 60-120 seconds, preferably 90 seconds. To this suspension freshly prepared solution of 0.1 M1-ethyl-3(3-dimethyl-amino-propyl) carbodimide hydrochloride (EDC) in 20mM MES buffer (pH 5.5) taken in the preferred ratio of 1:1, is added drop wise, while the solution is slowly vortexed. The tube is rotated slowly end-over-end for about 3 hours at a temperature of 20-25°C. It Is then washed thrice with 20mM MES buffer (pH 5.5) at 10,000 rpm for 10-12 minutes at a temperature of about 4°C. The latex particles are resuspended in MES buffer (20mM, pH5.5) and sonicated for 60-120 seconds by a tip sonicator at 5 watts.

### (c) Coating of Latex Particles with Antibody (immunoglobulins):

To the suspension of latex particles prepared in step (b), 0.6-1.0 mg preferably 0.8 mg per ml of the suspension, immunolobulins prepared in step (a) are added. The whole mixture is then rotated end-over-end for 18-20 hours at a temperature of 20-25°C. The coating reaction is stopped by addition of 1M glycine (pH 11.0) taken in quantity of 0.06ml per ml of solution of immunoglobulin coated latex particles. Rotation is continued for about 30 minutes at a temperature of 20-25°C. The coated latex particles are pelleted out by centrifugation at 10,000 rpm for 10-12 minutes at a temperature of about 4°C. The pellet is washed thrice with washing buffer (50mM glycine, pH8.5; 0.03% triton X-100 and 0.05% sodium azide) at 10,000 rpm for 10-12 minutes at a temperature of about 4°C. Finally the washed coated latex particles are resuspended in storage buffer (50mM glycine, pH8.5; 1.0% bovine serum albumin; 0.03% triton X-100; 0.1% sodium azide and 0.01% thiomersol) to a final concentration of 1% and sonicated by a tip sonicator for around 60 seconds at about 5 watts and stored at 4°C.

### METHOD OF USE

(a) Take 20-40µl (1 to 2 drops) of test serum, positive and negative controls at three distinct places on a glass slide
(b) Add 10-2µl (1-2 small drops) of latex reagent to test serum, positive and negative controls
(c) Mix the reactants with separate wooden sticks carefully to avoid any intermixing of reactant placed at separate places and rotate the slide for 1-2 minutes.

A positive reaction Is Indicated by the development of an agglutination within 1-2 minutes of mixing the reagent with the test sample and positive control, showing clearly visible clumping of the particles. The speed of appearance and quality of agglutination depends on the strength of the antigen present, varying from large clumps which appear within a few seconds of mixing, to small clumps which develop rather slowly. In negative reaction the reagent does not agglutinate and the cloudiness or the turbid nature remains substantially unchanged throughout the test.

Laboratory studies on the reliability of proposed agglutination reagent for rapid detection of Salmonella typhi in typhoid patient serum is performed with the laboratory strains of Salmonella typhi; and culture proven and widal positive serum samples collected from suspected cases of typhoid; and with serum samples of apparently normal healthy individuals. The result indicate 93.00% sensitivity and 98.00% specificity.

The present invention will now be illustrated with a working example which is intended to be illustrative example and is not intended to be taken restrictively to imply any limitation on the scope of the present invention.

### WORKING EXAMPLE

Flagellin gene sequence specific to salmonella typhi was amplified by polymerase chain reaction (PCR) using gene specific primers. Amplified PCR product was cloned in Glutathione-S-transferase (GST) vector and later expressed. The expressed protein was purified by GST affinity column chromatography. The protein content of the purified product was determined by Bradford method. Hyper immune serum against this protein was raised in rabbit. Immunoglobulins fraction of hyper immune sera was separated by ammonium sulphate precipitation. The precipitated immunoglobulins were suspended in 1.0 ml PB (50 mM, pH 7.2), dialysed and protein content determined. 1.0 ml of 1% carboxylated latex particles and 1.0 ml of 40 mM MES buffer (pH 5.5 - 6.5) were taken in 2.0 ml microcentrifuge tube. Then they were mixed on vortex mixer for 60 seconds and centrifuged at 10,000 RPM for 10-12 minutes at a temperature of 4°C. The latex particles were further washed twice in 2.0 ml of 20mM MES buffer (pH 5.5) by mixing on vortex mixer for 60 seconds and centrifugation at 10,000 RPM for 10-12 minutes at a temperature of 4°C. Following the final wash, the latex particles were suspended in 1.0 ml MES buffer (20mM, pH5.5) and sonicated by a tip sonicator at 5 watts for 60-120 seconds. Later 1.0 ml of freshly prepared solution of 0.1 M 1-ethyl-3-(3-dimethylaminopropyl) carbodimide hydrochloride (EDC) in MES buffer (20mM, pH 5.5) was added drop wise while the solution was slowly vortexed. Then the tube was rotated slowly end-over-end for 3 hours at a temperature of 20-25°C followed by washing three times with MES buffer (20mM, pH 5.5) at 10,000 RPM for 10-12 minutes at a temperature of 4°C. The latex particles were resuspended in 0.7 ml MES buffer (20mM,pH 5.5) and sonicated for 60-120 seconds by a tip sonicator at 5 watts. 0.8 mg of immunoglobulins were added to latex particles and volume was made up to 1.0 ml with MES buffer (20mM, pH 5.5). This was then rotated end-over-end for 18-20 hours at a temperature of 20-25°C. The coating reaction was then stopped by addition of 0.06 ml of 1M glycine (pH 11.0). The rotation was continued for 30 minutes at a temperature of 20-25°C. The coated latex particles were pelleted out by centrifugation at 10,000 RPM for 10-12 minutes at a temperature of 4°C. The pellet was washed thrice with 2.0 ml of washing buffer (50mM glycine, pH 8.5, 0.03% triton X-100 and 0.05% sodium azide) at 10,000 RPM for 10-12 minutes at a temperature of 4°C. The washed coated latex particles were resuspended In storage buffer (50mM glycine, pH 8.5, 1.0% bovine serum albumin, 0.03% triton X-100, 0.1% sodium azide and 0.01% thiomersol) to a final concentration of 1% and sonicated with tip sonicator for 60 seconds at 5 watts and stored at 4°C.

It is to be understood that the present invention is susceptible to modifications, changes and adaptations by those skilled In the art. Such modifications, changes, adaptations are intended to be within the scope of the present invention which is further set forth under the following claims:-

## Claims

1. Process for the preparation of an agglutination reagent for rapid and early detection of typhoid, comprising in the steps of:
(a) preparing Salmonella typhi specific antibody
(b) preparing latex particles suspension
(c) coating said latex particles with the antibody
**characterized in that** the antibody is prepared against the Flagellin gene sequence specific to Salmonella typhi and said Flagellin gene sequence specific to Salmonella typhi is cloned and expressed by recombinant DNA technology.

2. Process as claimed in claim 1 wherein an expressed recombinant protein is purified by affinity chromatography.

3. Process as claimed in claim 2 wherein a hyper immune sera against the recombinant protein is raised in rabbit.

4. Process as claimed in claim 3 wherein an immunoglobulin fraction of hyper immune sera is separated by ammonium sulphate precipitation.

5. Process as claimed in claim 4 wherein the precipitated immunoglobulins are suspended in phosphate buffer dialysed and the protein content is determined.

6. Process as claimed in claim 1 wherein 1% carboxylated latex particles of size 0.88 to 0.90 µm and 40 mM 40 2-N Morphilmoethane sulponic acid (MES) buffer of pH 5.5 to 6.0 are taken in a preferred ratio of 1:1.

7. Process as claimed in claim 6 wherein the suspension of latex particles in MES buffer is mixed on a vortex mixer for around 60 seconds and centrifuged at 10,000 rpm for 10-12 minutes at about 4°C.

8. Process as claimed in claim 7 wherein the latex particles are further washed twice with 20 mM MES buffer of pH 5.5 at 10,000 rpm for 10-12 minutes at about 4°C.

9. Process as claimed in claim 8 wherein the washed latex particles are suspended in 20mM MES buffer of pH 5.5 and the volume is made up as equal to the starting volume of latex particles.

10. Process as claimed in claim 9 wherein the suspended latex particles are sonicated by a tip sonicator at about 5 watts for 60-120 seconds preferably 90 seconds.

11. Process as claimed in claim 10 wherein freshly prepared solution of 0.1 M 1-ethyl-3 (3-dimethyl-amiuno propyl) carbodimide hydrochloride (EDC) in 20 mM MES buffer of pH 5.5, in a preferred ratio of 1:1 is added drop wise to the latex particles suspension, while the suspension is slowly vortexed.

12. Process as claimed in claim 11 wherein the latex particles with EDC solution are rotated slowly end-over-end for about 3 hours at a temperature of 20-25°C and washed thrice with 20 mM MES buffer (pH 5.5) at 10,000 rpm for 10-12 minutes at a temperature of about 4°C.

13. Process as claimed in claim 12 wherein the latex particles are resuspended in MES buffer (20 mm, pH 5.5) and sonicated by a tip sonicator for 60-120 seconds at about 5 watts.

14. Process as claimed in claims 4 and 13 wherein to the latex particle suspension 0.6-1.0 mg preferably 0.8 mg per ml of the immunoglobulins are added.

15. Process as claimed in claim 14 wherein the suspension of latex particles and immunoglobulins is rotated end-over-end for 18-20 hours at a temperature of about 20-25°C.

16. Process as claimed in claim 1 wherein the coating reaction is stopped by 1M glycine (pH 11.0) taken in quantity of 0.06 ml per ml of solution of immunoglobulin coated latex particles.

17. Process as claimed in claim 16 wherein the coated latex particles are pelleted out by centrifugation at 10,000 rpm for 10-12 minutes at a temperature of about 4°C.

18. Process as claimed in claim 17 wherein the pellet of coated latex particles is washed thrice with washing buffer comprising of 50 mM glycine, pH 8.5; 0.03% triton X-100 and 0.05% sodium azide; at 10,000 rpm for 10-12 minutes at a temperature of about 4°C.

19. Process as claimed in claim 18 wherein the washed and coated latex particles are suspended in storage buffer comprising of 50 mM glycine pH 8.5; 1.0% bovine serum albumin; 0.03% triton X-100; 0.1% sodium azide and 0.01% thiomersal, to a final concentration of 1%.

20. Process as claimed in claim 19 wherein the 1% suspension of coated latex particles is sonicated by a tip sonicator for around 60 seconds at about 5 watts and stored at 4°C.

21. Process as claimed in claim 20, wherein 1% carboxylated latex particles of size 0,88-0,90µ are coated with antibody specific to Salmonella typhi, suspended in storage buffer by adding 0,6-1,0 mg immoglobulines per ml of the suspension.

22. Agglutination reagent as prepared by the process as claimed in claim 1.

23. Agglutination reagent as claimed in claim 22 wherein 1% carboxylated latex particles of size 0.88 to 0.90 µm and 40 mM 2-N Morphilinoethane sulphonic acid MES buffer (pH 5,5-6,0) are taken in a preferred ratio of 1:1.

24. Agglutination reagent as claimed in claim 22 wherein the coated latex particles are resuspended in a storage buffer comprising 50 mM glycine, pH 8.5, 1.0% bovine serum albumin, 0.03% triton X-100, 0.1 % sodium azide and 0.01 % thiomersol to a final concentration of 1%.

25. Agglutination reagent as claimed in claim 22 wherein the said antibody is the immunoglobulin fraction of the hyper immune sera raised in rabbit against the recombinant protein expressed by cloning of Flagellin gene sequence specific to Salmonella typhi by recombinant DNA technology, suspended in 50 mM phosphate buffer.

## Patentansprüche

1. Verfahren zur Herstellung eines Agglutiationsreagenz zum schnellen und frühen Erkennen von Typhus, beinhaltend die Schritte:
a) Herstellen eines Salmonella typhi spezifischen Antikörpers;
b) Herstellen einer Latexpartikel-Suspension;
c) Beschichten der Latexpartikel mit dem Antikörper;
**dadurch gekennzeichnet,**
**dass** der Antikörper gegen die Salmonella typhi eigene Flagellin-Gensequenz hergestellt wird und dass die Salmonella typhi eigene Flagelin-Gensequenz geklont und durch Rekombinations-DNA-Technologie exprimiert wird.

2. Verfahren nach Anspruch 1,
**dadurch gekennzeichnet,**
**dass** ein exprimiertes Rekombinationsprotein durch Affinitätschromatographie gereinigt wird.

3. Verfahren nach Anspruch 2,
**dadurch gekennzeichnet,**
**dass** ein Hyperimmunserum gegen das Rekombinationsprotein in Kaninchen erhalten oder gezüchtet wird.

4. Verfahren nach Anspruch 3,
**dadurch gekennzeichnet,**
**dass** eine Immunglobulin-Fraktion des Hyperimmunserums durch Ammoniumsulfat-Ausfällung abgeschieden wird.

5. Verfahren nach Anspruch 4,
**dadurch gekennzeichnet,**
**dass** die ausgefällten Immunglobuline in einem dialysierten Phosphatpuffer suspendiert werden und dass der Proteingehalt bestimmt wird.

6. Verfahren nach Anspruch 1,
**dadurch gekennzeichnet,**
**dass** 1% carboxylierter Latexpartikel der Größe 0,88 bis 0,90 µm und 40 mM 2-N-Morpholinoethansulfonsäure (MES)-Puffer mit pH 5,5 bis 6,0 in einem bevorzugten Verhältnis von 1:1 verwendet werden.

7. Verfahren nach Anspruch 6,
**dadurch gekennzeichnet,**
**dass** die Suspension von Latexpartikeln in MES-Puffer auf einem Vortexmischer für etwa 60 s gemischt und bei 10.000 U/min während 10 - 12 min bei etwa 4°C zentrifugiert wird.

8. Verfahren nach Anspruch 7,
**dadurch gekennzeichnet,**
**dass** die Latexpartikel weiterhin zweimal mit 20 mM MES-Puffer bei pH 5,5 bei 10.000 U/min während 10 - 12 min bei etwa 4°C gewaschen werden.

9. Verfahren nach Anspruch 8,
**dadurch gekennzeichnet,**
**dass** die gewaschenen Latexpartikel in 20 mM MES-Puffer mit pH 5,5 suspendiert werden und dass das Volumen so eingestellt wird, dass es dem Anfangsvolumen der Latexpartikel entspricht.

10. Verfahren nach Anspruch 9,
**dadurch gekennzeichnet,**
**dass** die in Suspension befindlichen Latexpartikel mit einem Spitzen-Sonicator bei etwa 5 Watt während 60 - 120 s, vorzugsweise 90 s, sonifiziert werden.

11. Verfahren nach Anspruch 10,
**dadurch gekennzeichnet,**
**dass** eine frisch zubereitete Lösung von 0,1 M 1-Ethyl-3-(3-Dimethylaminopropyl-)Carbodiimid-Hydrochlorid (EDC) in 20mM MES-Puffer mit pH 5,5 in einem bevorzugten Verhältnis von 1:1 tropfenweise der Latexpartikel-Suspension zugegeben wird, während die Suspension langsam verwirbelt wird.

12. Verfahren nach Anspruch 11,
**dadurch gekennzeichnet,**
**dass** die Latexpartikel mit der EDC-Lösung langsam etwa 3 h lang end-over-end bei einer Temperatur von 20-25°C gedreht und dreimal mit 20 mM MES-Puffer (pH 5,5) bei 10.000 U/min während 10 - 12 min bei einer Temperatur von ungefähr 4°C gewaschen werden.

13. Verfahren nach Anspruch 12,
**dadurch gekennzeichnet,**
**dass** die Latexpartikel erneut in MES-Puffer (20 mM, pH 5,5) verteilt bzw. aufgeschlämmt und mit einem Spitzen-Sonicator während 60 bis 120 s bei etwa 5 Watt sonifiziert werden.

14. Verfahren nach Anspruch 4 und Anspruch 13,
**dadurch gekennzeichnet,**
**dass** pro ml der Latexpartikel-Suspension 0,6 - 1,0 mg, vorzugsweise 0,8 mg der Immunglobuline zugegeben werden.

15. Verfahren nach Anspruch 14,
**dadurch gekennzeichnet,**
**dass** die Suspension aus Latexpartikeln und Immunglobulinen während 18-20 h bei einer Temperatur von etwa 20 - 25°C end-over-end gedreht wird.

16. Verfahren nach Anspruch 1,
**dadurch gekennzeichnet,**
**dass** die Beschichtungsreaktion durch 1 M Glycin (pH 11,0) gestoppt wird, das in einer Menge von 0,06 ml pro ml der Lösung aus mit Immunglobulin beschichteten Latexpartikeln verwendet wird.

17. Verfahren nach Anspruch 16,
**dadurch gekennzeichnet,**
**dass** die beschichteten Latexpartikel als Pellet-Niederschlag durch Zentrifugieren bei 10.000 U/min während 10 - 12 min bei der Temperatur von etwa 4°C abgeschieden werden.

18. Verfahren nach Anspruch 17,
**dadurch gekennzeichnet,**
**dass** der Pellet-Niederschlag aus beschichteten Latexpartikeln dreimal mit einem Waschpuffer mit 50 mM Glycin, pH 8,5; 0,03% Triton X-100 und 0,05% Natriumazid bei 10.000 U/min während 10 - 12 min bei einer Temperatur von ungefähr 4° gewaschen wird.

19. Verfahren nach Anspruch 18,
**dadurch gekennzeichnet,**
**dass** die gewaschenen und beschichteten Latexpartikel in einem Vorratspuffer mit 50 mM Glycin pH 8,5; 1,0% bovines Serum-Albumin; 0,03% Triton X-100; 0,1% Natriumazid und 0,01% Thiomersol bis zu einer endgültigen Konzentration von 1% suspendiert werden.

20. Verfahren nach Anspruch 19,
**dadurch gekennzeichnet, dass** die 1%-Suspension beschichteter Latexpartikel mit einem Spitzen-Sonicator während etwa 60 s bei etwa 5 Watt sonifiziert und bei 4°C gelagert wird.

21. Verfahren nach Anspruch 20,
**dadurch gekennzeichnet,**
**dass** 1% carboxylierter Latexpartikel mit einer Größe von 0,88 - 0,90 µm, die in dem Vorratspuffer suspendiert sind, mit einem Salmonella typhi eigenen Antikörper beschichtet werden, indem 0,6 - 1,0 mg Immunglobuline pro ml der Suspension zugegeben werden.

22. Aggluniationsreagenz, das nach dem Verfahren gemäß Anspruch 1 hergestellt ist.

23. Agglunationsreagenz gemäß Anspruch 22,
**dadurch gekennzeichnet,**
**dass** 1% carboxylierte Latexpartikel der Größe 0,88 bis 0,90 µm und 40 mM 2-N-Morpholinoethansulfonsäure (MES)-Puffer (pH 5,5-6,0) in einem bevorzugten Verhältnis von 1:1 verwendet werden.

24. Aggluniationsreagenz nach Anspruch 22,
**dadurch gekennzeichnet,**
**dass** die beschichteten Latexpartikel in einem Vorratspuffer mit 50 mM Glycin, pH 8,5, 1,0% bovines Serum-Albumin, 0,03% Triton X-100, 0,1% Natriumazid und 0,01% Thiomersol bis zu einer endgültigen Konzentration von 1% erneut suspendiert werden.

25. Aggluniationsreagenz nach Anspruch 22,
**dadurch gekennzeichnet,**
**dass** der Antikörper die Immunglobulin-Fraktion des Hyperimmunserums ist, das gegen das Rekombinationsprotein in Kaninchen erhalten oder gezüchtet wurde, die **dadurch** exprimiert wurde, dass die Salmonella typhi eigene Flagellin-Gensequenz durch Rekombinations-DNA-Technologie geklont wurde, und die in 50 mM Phosphatpuffer suspendiert ist.

## Revendications

1. Procédé de préparation d'un réactif d'agglutination destiné à la détection rapide et précoce de la typhoïde, comprenant les étapes:
(a) de préparation de l'anticorps spécifique de la Salmonella Typhimurium
(b) de préparation de la suspension des particules de latex
(c) d'enduction desdites particules de latex avec l'anticorps **caractérisé en ce que** l'anticorps est préparé par rapport à la séquence du gène de la flagelline spécifique de la Salmonella Typhimurium et que ladite séquence du gène de la flagelline spécifique à la Salmonella Typhimurium est clonée et exprimée par la technologie d'ADN recombinant.

2. Procédé selon la revendication 1 dans lequel une protéine recombinante exprimée est purifiée par chromatographie d'affinité.

3. Procédé selon la revendication 2 dans lequel un sérum hyperimmun contre la protéine recombinante est obtenu ou élevé chez le lapin,

4. Procédé selon la revendication 3 dans lequel une fraction d'immunoglobuline du sérum hyperimmun est séparée par précipitation au sulfate d'ammonium.

5. Procédé selon la revendication 4 dans lequel les immunoglobulines précipitées sont mises en suspension dans un tampon phosphate dialysé et la teneur en protéine est déterminée.

6. Procédé selon la revendication 1 dans lequel des particules de latex carboxylées à 1% de taille 0,88-0,90 µm et un tampon d'acide 2-(N-morpholino)éthane sulfonique (MES) 40 mM de pH 5,5-6,0 sont utilisés de préférence dans un rapport de 1:1.

7. Procédé selon la revendication 6 dans lequel la suspension des particules de latex dans le tampon MES est mélangée sur un agitateur vortex pendant environ 60 secondes et centrifugée à 10 000 tr/min pendant 10-12 minutes à environ 4 °C.

8. Procédé selon la revendication 7 dans lequel les particules de latex sont, en outre, lavées deux fois avec un tampon MES 20 mM de pH 5,5 à 10 000 tr/min pendant 10-12 minutes à environ 4 °C.

9. Procédé selon la revendication 8 dans lequel les particules de latex lavées sont suspendues dans un tampon MES 20 mM de pH 5,5 et le volume est complété afin d'être égal au volume initial des particules de latex.

10. Procédé selon la revendication 9 dans lequel les particules de latex en suspension sont soumises à une sonication par une pointe émettrice d'ultrasons à environ 5 watts pendant 60-120 secondes, de préférence 90 secondes.

11. Procédé selon la revendication 10 dans lequel une solution fraîchement préparée de chlorhydrate de 1-éthyl-3-(3-diméthylaminopropyl)carbodiimide (EDC) 0,1 M dans un tampon MES 20 mM de pH 5,5, dans un ratio de préférence de 1:1, est ajoutée goutte à goutte à la suspension des particules de latex, pendant que la suspension est lentement agitée sur vortex.

12. Procédé selon la revendication 11 dans lequel les particules de latex avec la solution d'EDC sont mises en rotation, lentement et de manière verticale, pendant environ 3 heures à une température de 20-25 °C, et sont lavées trois fois avec du tampon MES 20 mM de pH 5,5 à 10 000 tr/min pendant 10-12 minutes à environ 4 °C.

13. Procédé selon la revendication 12 dans lequel les particules de latex sont remises en suspension dans un tampon MES (20 mM, pH 5,5) et soumises à une sonication par une pointe émettrice d'ultrasons pendant 60-120 secondes à environ 5 watts.

14. Procédé selon les revendications 4 et 13 dans lequel 0,6-1,0 mg, de préférence 0,8 mg par ml d'immunoglobulines sont ajoutées à la suspension de particules de latex.

15. Procédé selon la revendication 14 dans lequel la suspension des particules de latex et d'immunoglobulines est mise en rotation de manière verticale pendant 18-20 heures à une température d'environ 20-25 °C.

16. Procédé selon la revendication 1 dans lequel la réaction d'enduction est arrêtée par de la glycine 1M (pH 11,0), utilisée dans une quantité de 0,06 ml par ml de solution de particules de latex enduites d'immunoglobuline.

17. Procédé selon la revendication 16 dans lequel les particules de latex enduites sont pastillées par centrifugation à 10 000 tr/min pendant 10-12 minutes à une température d'environ 4 °C.

18. Procédé selon la revendication 17 dans lequel la pastille de particules de latex enduites est lavée trois fois avec un tampon de lavage constitué de glycine 50 mM, pH 8,5; de triton X-100 à 0,03% et d'azide de sodium à 0,05%; à 10 000 tr/min pendant 10-12 minutes à une température d'environ 4 °C.

19. Procédé selon la revendication 18 dans lequel les particules de latex lavées et enduites sont mises en suspension dans un tampon de stockage constitué de glycine 50 mM pH 8,5; de sérum-albumine bovin à 1,0%; de triton X-100 à 0,03% ; d'azide de sodium à 0,1% et de thimérosal à 0,01% à une concentration finale de 1%.

20. Procédé selon la revendication 19 dans lequel la suspension de particules de latex enduites à 1% est soumise à une sonication par une pointe émettrice d'ultrasons pendant 60 secondes à environ 5 watts et stockée à 4 °C.

21. Procédé selon la revendication 20 dans lequel les particules de latex carboxylées à 1%, de taille 0,88-0,90 µm, sont enduites ou revêtues avec l'anticorps spécifique de la Salmonella Typhimurium, mises en suspension dans un tampon de stockage en ajoutant 0,6 - 1,0 mg d'immunoglobulines par ml de la suspension.

22. Réactif d'agglutination préparé tel que dans le procédé selon la revendication 1.

23. Réactif d'agglutination selon la revendication 22 dans lequel les particules de latex carboxylées à 1%, de taille 0,88-0,90 µm, et un tampon d'acide 2-(N-morpholino)éthane sulfonique MES 40 mM (pH de 5,5 à 6,0) sont utilisés de préférence dans un rapport de 1:1.

24. Réactif d'agglutination selon la revendication 22 dans lequel les particules de latex enduites ou recouvertes sont remises en suspension dans un tampon de stockage constitué de glycine 50 mM, pH 8,5; de sérum-albumine bovin à 1,0%; de triton X-100 à 0,03%; d'azide de sodium à 0,1% et de thimérosal à 0,01% à une concentration finale de 1%.

25. Réactif d'agglutination selon la revendication 22 dans lequel ledit anticorps est la fraction d'immunoglobuline du sérum hyperimmun obtenu ou élevé chez le lapin contre la protéine recombinante exprimée par clonage de la séquence du gène de la flagelline spécifique à la Salmonella Typhimurium par technologie d'ADN recombinant, mise en suspension dans un tampon phosphate 50 mM.
